# EUROPEAN PATENT SPECIFICATION

(11) **EP 3 041 365 B1**
(45) Date of publication and mention of the grant of the patent: **04.04.2018**
(21) Application number: 14749752.3
(22) Date of filing: 31.07.2014
(51) Int. Cl.: A23K 10/40, A23K 50/00

(54) **USE OF A FEED COMPOSITION FOR REDUCING METHANE EMISSION IN RU-MINANTS, AND/OR TO IMPROVE RUMINANT PERFORMANCE**
VERWENDUNG EINER FUTTERZUSAMMENSETZUNG ZUR REDUKTION DER METHANEMISSION VON WIEDERKÄUERN UND/ODER ZUR VERBESSERUNG DER WIEDERKÄUERLEISTUNG
UTILISATION D'UNE COMPOSITION ALIMENTAIRE POUR ANIMAUX POUR RÉDUIRE LES ÉMISSIONS DE MÉTHANE CHEZ LES RUMINANTS, ET/OU AMÉLIORER LE RENDEMENT DES RUMINANTS

(30) Priority: 07.08.2013 EP 13179546
(43) Date of publication of application: 13.07.2016
(73) Proprietor: DSM IP Assets B.V., 6411 TE Heerlen (NL)
(72) Inventor: DUVAL, Stephane, CH-4303 Kaiseraugst (CH); KINDERMANN, Maik, CH-4303 Kaiseraugst (CH)
(74) Representative: Rabanus, Birgit
(86) International application number: PCT/EP2014/066459
(87) International publication number: WO 2015/018726

(56) References cited:
- WO-A1-2011/070133
- WO-A1-2012/084629
- WO-A2-2012/160191
- Anonymous: "Material Safety Data Sheet 4-Nitroaniline MSDS", ScienceLab.com , 10 October 2005 (2005-10-10), XP002713228, Retrieved from the Internet: URL:http://www.sciencelab.com/msds.php?msd sId=9927373 [retrieved on 2013-09-19]

## Description

The present invention relates to the field of reduction of methane emission in ruminants. Particularly, it relates to the use of a feed composition or a feed additive comprising at least one para nitroaniline derivative and at least one organic molecule substituted at any position with at least one nitrooxy group, for reducing the production of methane emanating from the digestive activities of ruminants, and/or to improve the ruminant performance.

The present invention further relates to animal feed or animal feed compositions and feed additives comprising the above mentioned molecules. The term feed or feed composition means any compound, preparation, mixture, or composition suitable for, or intended for intake by an animal.

In the present context, a ruminant is a mammal of the order Artiodactyla that digests plant-based food by initially softening it within the animal's first stomach, known as the rumen, then regurgitating the semi-digested mass, now known as cud, and chewing it again. The process of again chewing the cud to further break down plant matter and stimulate digestion is called "ruminating".

Rumen fermentation brings some disadvantages. Methane is produced as a natural consequence of the anaerobic fermentation, which represents an energy loss to the host animal. Carbohydrate makes up 70 - 80% of the dry matter in a typical dairy cattle ration and in spite of this the absorption of carbohydrates from the gastrointestinal tract is normally very limited. The reason for this is the extensive fermentation of carbohydrates in the rumen resulting in production of acetate, propionate and butyrate as the main products. These products are part of the so called volatile fatty acids, (VFAs).

Besides the energy loss, methane is also a greenhouse gas, which is many times more potent than CO₂. Its concentration in the atmosphere has doubled over the last century and continues to increase alarmingly. Ruminants are the major contributors to the biogenic methane formation, and it has been estimated that the prevention of methane formation from ruminants would almost stabilize atmospheric methane concentrations.

Bile acid derivatives leading to reduction of methane emission, when tested using an *in vitro* rumen simulation model, have recently been published (WO 2010/072584). However, the amount required to produce a moderate reduction of methane emission is not compatible with the ruminant feed industry cost constraints.

Furthermore, a number of natural plant extracts (Garlic: WO 2009150264, yucca, cinnamon, rhubarb...) have been described in the scientific literature as potent solutions to reduce methane emission in ruminants based on *in vitro* experiments. However, none of these solutions made it to a commercial product because of side effects (residues in milk), because of lack efficacy, when tested *in vivo,* or because of the very large amount of additive which needs to be supplied to the animal to generate a significant methane reduction.

Nitrooxy organic molecules were recently shown to exhibit significant methane reduction effect in vitro, and when fed to ruminants (WO2012084629). However, these compounds targeting a single mechanism may ultimately lead with time in application to development of resistance, thereby loosing efficiency with time. Under these circumstances there is still a need to develop new compositions which reduce the formation of methane produced by ruminants and which effect persists over time. In addition to reducing methane emission, such compositions may also contribute to improve ruminant performance by improving the feed conversion ratio, reducing feed intake, improving weight gain, and/or improving carcass, or milk yield.

The present inventors now surprisingly found that the composition specified herein after, have a great potential for use in animal feed in order to essentially reduce the formation of methane in a persistent way, without affecting microbial fermentation in a way that would be detrimental to the host animal. Moreover, the composition of the present invention also have a great benefit regarding overall animal performance as measured by ruminal acetate/propionate ratio, feed conversion ratio, feed intake, weight gain, carcass yield, or milk yield. Said compositions are also more stable than those described in the prior art, safer for the animal and human, lead to persistent methane reduction effect, they do not affect palatability, they can be produced at industrial scale at a cost compatible with the animal nutrition industry, and above all, they do not provoke accumulation of any metabolite in the milk or meat of the supplemented animal, and they are active at very low concentration in the rumen due to a synergistic effect.

Therefore, the present invention provides the use of a feed composition or feed additive comprising at least one para nitroaniline derivative or a salt thereof as defined by formula (I), and at least one organic molecule substituted at any position with at least one nitrooxy group, or a salt thereof as defined by formula III for reducing the formation of methane emanating from the digestive activities of ruminants and/or for improving ruminant performance.

The invention also provides a feed composition or a feed additive comprising at least one para nitroaniline derivative or a salt thereof as defined in formula (I) and at least one organic molecule or a salt thereof of formula III as defined below.

The invention further provides a method for reducing the production of methane emanating from the digestive activities of ruminants and/or for improving ruminant animal performance, comprising orally administering to the animal a sufficient amount of a feed composition or feed additive comprising at least one para nitroaniline derivative or salts thereof as defined by formula (I), and at least one organic molecule substituted at any position with at least one nitrooxy group, or salts thereof as defined by formula III to the animal. The term "oral administration" as used herein refers to simple feeding, or manual administration of a bolus. Disclosed herein is the use of a feed composition or feed additive comprising at least one para nitroaniline derivative or a salt thereof as defined by formula (I), 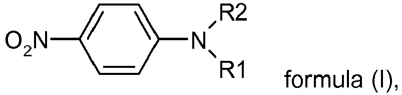 wherein R1 and R2 independently of each other represent H, -CH₃, or -CH₂R3, and wherein R3 is a saturated or unsaturated, linear, branched or cyclic C₁-C₈-hydrocarbon group, optionally substituted with 1 to 3 groups selected from -OH, - NH₂, -COOH, and wherein one or two of the carbon atoms in the C₁-C₈ hydrocarbon is optionally substituted by a nitrogen or an oxygen atom (in the following referred to as the "nitroaniline compound", and at least one organic molecule substituted at any position with at least one nitrooxy group, or a salt thereof as defined by formula (II) 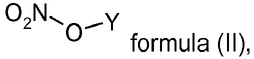 wherein Y is an organic molecule of the following composition: CₐH_{b}O_{d}NₑS_{g}, wherein
- a: is comprised between 1 and 25,
- b: is comprised between 2 and 51,
- d: is comprised between 0 and 8,
- e: is comprised between 0 and 5,
- g: is comprised between 0 and 3,
in the following referred to as the "nitrooxy compound", for reducing the formation of methane emanating from the digestive activities of ruminants and/or for improving ruminant performance.

In all embodiments of the present invention, preferred compounds of formula (I) according to the present invention are compounds, wherein R1 is H or -CH₃. More preferred compounds of formula (I) are compounds wherein R1 is H or -CH₃, R2 is H, -CH₃, or -CH₂R3, and R3 is a saturated or unsaturated, linear, or branched C₁-C₄ -hydrocarbon group. Even more preferred compounds of formula (I) are compounds wherein R1 is H or -CH₃, R2 is H, -CH₃, or -CH₂R3, and R3 is a saturated, linear, or branched C₁-C₃-hydrocarbon group.

Most preferred compounds of formula (I) according to the present invention are selected from the group of compounds comprising (4-nitro-phenyl)-amine, methyl-(4-nitro-phenyl)-amine, ethyl-(4-nitro-phenyl)-amine, dimethyl-(4-nitro-phenyl)-amine, (4-Nitro-phenyl)-propyl-amine, isopropyl-(4-nitro-phenyl)-amine, 2-(4-Nitro-phenylamino)-ethanol, 3-(4-Nitro-phenylamino)-propan-1-ol, 4-Nitro-N-(Pyridin-2-ylmethyl)aniline, 4-((4-Nitro-phenylamino)-methyl)-benzoic acid, and 3-((4-Nitro-phenylamino)-methyl)-benzoic acid as listed and depicted in table 1. Even most preferred compound of formula (I) according to the present invention is (4-nitro-phenyl)-amine.

**Table 1: Preferred compounds of formula (I) according to the present invention**

| **Compound Identifier** | **Compound structure** | **Compound name** |
|---|---|---|
| 1 | 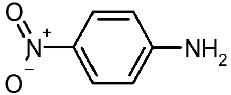 | (4-nitro-phenyl)-amine |
| 2 | 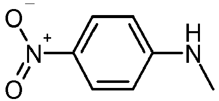 | Methyl-(4-nitro-phenyl)-amine |
| 3 | 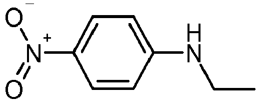 | Ethyl-(4-nitro-phenyl)-amine |
| 4 | 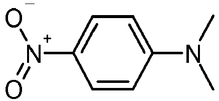 | Dimethyl-(4-nitro-phenyl)-amine |
| 5 | 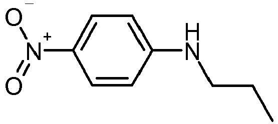 | (4-Nitro-phenyl)-propylamine |
| 6 | 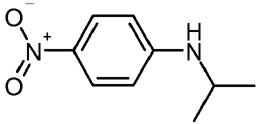 | Isopropyl-(4-nitro-phenyl)-amine |
| 7 | 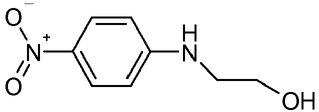 | 2-(4-Nitro-phenylamino)-ethanol |
| 8 | 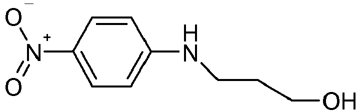 | 3-(4-Nitro-phenylamino)-propan-1-ol |
| 9 | 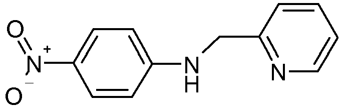 | 4-Nitro-N-(Pyridin-2-ylmethyl)aniline |
| 10 | 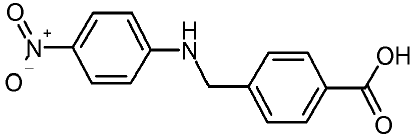 | 4-((4-Nitro-phenylamino)-methyl)-benzoic acid |
| 11 | 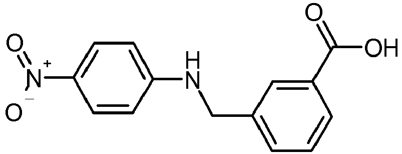 | 3-((4-Nitro-phenylamino)-methyl)-benzoic acid |

For all embodiments of the present invention, it is to be understood that compounds of formula (I) can be in any isomeric form.

The compounds of formula (I) of the present invention may also be in the form of a salt. Preferred cations for salt preparation may be selected from the group consisting of sodium (Na+), potassium (K+), lithium (Li+), magnesium (Mg2+), calcium (Ca2+), barium (Ba2+), strontium (Sr2+), and ammonium (NH4+).

The compounds of formula (I) according to the present invention can be manufactured in principle according to synthetic methods known *per se* in the art, and/or based on methods as described in PCT/IB2013052230.

In all these cases appropriate methods to purify the product (compounds of formula (I)) can be chosen by those skilled in the art, i.e. by column chromatography, or the compound of formula (I), can be isolated and purified by methods known per se.

In all embodiments of the present invention, the compounds defined by formula (II) are obviously limited to the compounds that are chemically plausible. The person skilled in the art knows that if e.g.: a is 1, d, e, and g are 0, then, b needs to be 3; similarly, if a is 2, d, e, and g are 0, then, b needs to be 5, or if a is 3, d, e, and g are 0, then, b needs to be 7, or if a is 1, d is 1, e, and g are 0, then, b can either be 2 or 3 etc. Disclosed herein are organic molecules substituted at any position with at least one nitrooxy group, or salts thereof are compounds of formula (II), 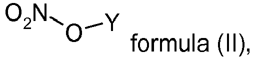 wherein Y is an organic molecule of the following composition: CₐH_{b}O_{d}NₑS_{g}, wherein
- a: is comprised between 1 and 10
- b: is comprised between 2 and 21
- d: is comprised between 0 and 6
- e: is comprised between 0 and 3
- g: is comprised between 0 and 1.
Compounds of formula (II) according to the composition of the present invention are compounds of formula (III), 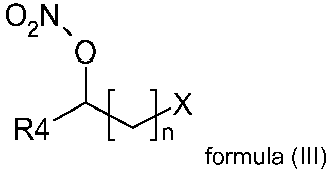 wherein
- n: is comprised between 0 and 12, preferably comprised between 0 and 6 and, wherein, if n ≠ 0, the carbon chain is a linear, a cyclic, or branched aliphatic carbon chain which may be non substituted or substituted with up to 3 hydroxyl-, alkoxy-, amino-, alkylamino-, dialkylamino- or nitrooxy groups, or an alkenyl, or an alkynyl carbon chain mono- or polyunsaturated and in any isomeric form,
- R4: is independently, hydrogen or a saturated straight, cyclic or branched chain of an alkyl or alkenyl group containing 1 to 12, preferably 1 to 6 carbon atoms,
- X: is hydrogen, R5, R5≡N, -OR5, -OCOR5, -NR5R6, -ONO2, -COOR5, - CONR5R6, -NHSO2R5, or -SO2NHR5,
- R5 and R6: are independently, hydrogen, C1-C12 straight, branched or cyclic alkyl chain, non-substituted or substituted with up to 3 hydroxyl-, alkoxy-, amino-, alkylamino-, dialkylamino- or nitrooxy groups, alkenyl, or alkynyl carbon chain which may be mono or polyunsaturated, and in any isomeric form.

For all embodiments of the present invention, it is to be understood that compounds of formula (III) can be in any isomeric form.

It is to be understood in the above definition of compounds of formula (III) that when n > 2, the carbon chain can be linear or branched at any position along the carbon chain. In addition, the carbon chain can be branched by multiple branches at different positions along the carbon chain. Moreover, when n > 3, the aliphatic carbon chain may form a cyclic moiety. This cyclic moiety can carry the nitrooxy moiety at any position (2, 3, 4), and it can also be branched at multiple positions by any aliphatic groups. The branched aliphatic groups are preferably, methyl, ethyl or propyl. Moreover, the carbon chain may be further substituted with up to 3 hydroxyl-, alkoxy-, amino-, alkylamino-, dialkylamino- or nitrooxy groups.

In the above definition of derivatives of the formula (III) a preferred R4 alkyl group is methyl, ethyl, propyl, isopropyl, butyl, sec. butyl, isobutyl, pentyl, neopentyl, hexyl, cyclohexyl, and 2-ethyl-hexyl and octyl. Furthermore any alkyl or alkenyl group containing three or more carbon atoms can be straight chain, branched, or cyclic. In addition for the straight chain or branched C₂-C₁₀-alkenylene group, this is understood to encompass alkenylene groups with one or (from C₄) more double bonds; examples of such alkenylene groups are those of the formulae -CH=CH-, -CH=CH-CH₂-, -CH=CH-(CH₂)₃- and -(CH=CH)₂-. Compounds of formula (II) disclosed herein and salts thereof are selected from 3-Nitrooxypropanol, racemate-4-Phenylbutane-1,2-diyl dinitrate, 2-(Hydroxymethyl)-2-(nitrooxymethyl)-1,3-propanediol, N-Ethyl-3-nitrooxy-propionic sulfonyl amide, 5-Nitrooxy-pentanenitrile, 5-Nitrooxy-pentane, 3-Nitrooxy-propyl propionate, 1,3-bis-Nitrooxypropane, 1,4-bis-Nitrooxybutane, 1,5-bis-Nitrooxypentane, 3-Nitrooxy-propyl benzoate, 3-Nitrooxy-propyl hexanoate, 3-Nitrooxy-propyl 5-nitrooxy-hexanoate, Benzylnitrate, isosorbid-dinitrate, N-[2-(Nitrooxy)ethyl]-3-pyridinecarboxamide, 2-Nitro-5-nitrooxymethyl-furan, Bis-(2-nitrooxyethyl) ether, 3-nitrooxy propionic acid, methyl-3-nitrooxy propionate, Ethyl-3-nitrooxy propionate, Ethyl-4-nitrooxy butanoate, Ethyl-3-nitrooxy butanoate, 5-nitrooxy pentanoic acid, Ethyl-5-nitrooxy pentanoate, 6-nitrooxy hexanoic acid, Ethyl-6-nitrooxy hexanoate, ethyl-4-nitrooxy-cyclohexylcarboxylate, 8-nitrooxy octanoic acid, Ethyl-8-nitrooxy octanoate, 11-nitrooxy undecanoic acid, Ethyl-11-nitrooxy undecanoate, 5-nitrooxy-pentanoic amide, 5-nitrooxy-N-methyl-pentanoic amide, and salts thereof as listed with their chemical formula in Table 2. Compounds of formula (II) and slats thereof disclosed herein are selected from 3-Nitrooxypropanol, 5-Nitrooxy-pentane, 1,3-bis-Nitrooxypropane, ethyl-3-nitrooxy propionate, methyl-3-nitrooxy propionate, 3-nitrooxy propionic acid, 1,4-bis-Nitrooxybutane, and 1,5-bis-Nitrooxypentane. A compound of formula (II) and salt thereof is 3-Nitrooxypropanol.

**Table 2: Preferred compounds of formula (II)**

| **Comp. Identifier** | **Molecular structure** | **Chemical name** |
|---|---|---|
| A | 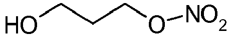 | 3-Nitrooxypropanol |
| B | 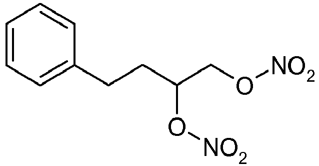 | rac-4-Phenylbutane-1,2-diyl dinitrate |
| C | 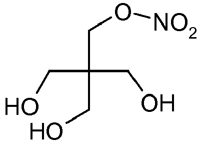 | 2-(Hydroxymethyl)-2-(nitrooxymethyl)-1,3-propanediol |
| D | 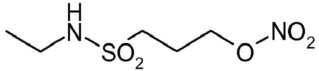 | N-Ethyl-3-nitro-oxy-propionic sulfonyl amide |
| E | 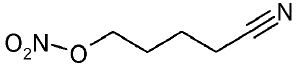 | 5-Nitrooxy-pentanenitrile |
| F | 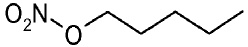 | 5-Nitrooxy-pentane |
| G | 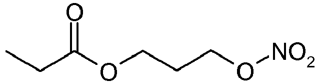 | 3-Nitro-oxy-propyl propionate |
| H | 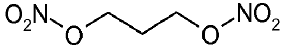 | 1,3-bis-Nitrooxypropane |
| I | 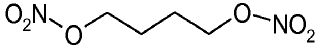 | 1,4-bis-Nitrooxybutane |
| J | 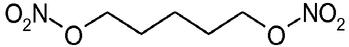 | 1,5-bis-Nitrooxypentane |
| K | 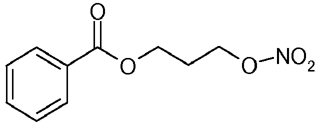 | 3-Nitro-oxy-propyl benzoate |
| L | 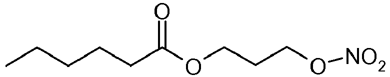 | 3-Nitro-oxy-propyl hexanoate |
| M | 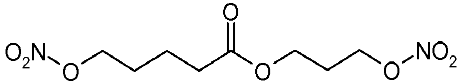 | 3-Nitro-oxy-propyl 5-nitro-oxy-hexanoate |
| N | 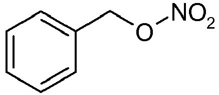 | Benzylnitrate |
| O | 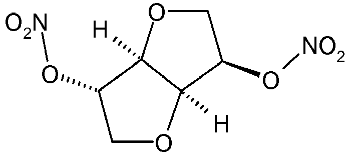 | isosorbid-dinitrate |
| P | 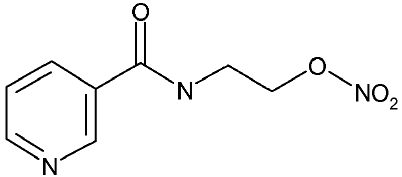 | N-[2-(N itrooxy)ethyl]-3-pyridinecarboxamide |
| Q | 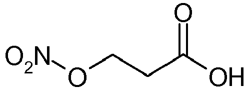 | 3-nitrooxy propionic acid |
| R | 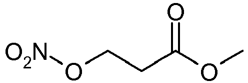 | methyl-3-nitrooxy propionate |
| S | 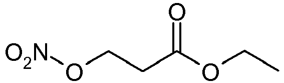 | Ethyl-3-nitrooxy propionate |
| T | 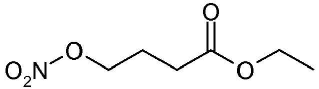 | Ethyl-4-nitrooxy butanoate |
| U | 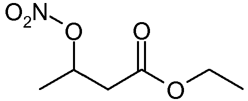 | Ethyl-3-nitrooxy butanoate |
| V | 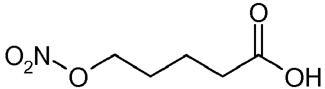 | 5-nitrooxy pentanoic acid |
| W | 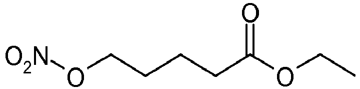 | Ethyl-5-nitrooxy pentanoate |
| X | 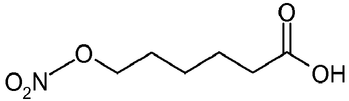 | 6-nitrooxy hexanoic acid |
| Y | 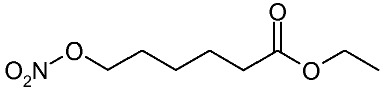 | Ethyl-6-nitrooxy hexanoate |
| Z | 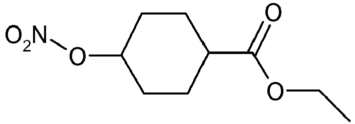 | ethyl-4-nitrooxy-cyclohexylcarboxylate |
| (AA) | 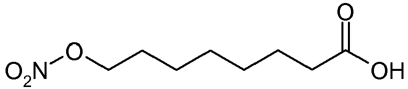 | 8-nitrooxy octanoic acid |
| (AB) | 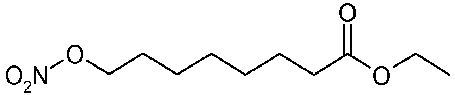 | Ethyl-8-nitrooxy octanoate |
| (AC) | 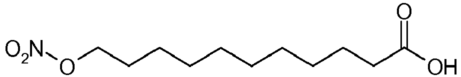 | 11-nitrooxy undecanoic acid |
| (AD) | 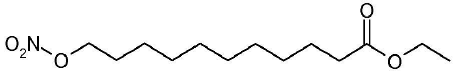 | Ethyl-11-nitrooxy undecanoate |
| (AE) | 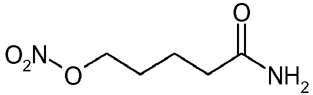 | 5-nitrooxy-pentanoic amide |
| (AF) | 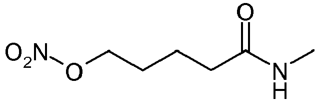 | 5-nitrooxy-N-methyl-pentanoic amide |

The compounds of formula (II) may also be present in the form of salts of the nitrooxy organic molecule. Preferred cations for salt preparation may be selected from the group consisting of sodium (Na+), potassium (K+), lithium (Li+), magnesium (Mg2+), calcium (Ca2+), barium (Ba2+), strontium (Sr2+), and ammonium (NH4+).

The compounds of formula (II), and (III) according to the composition of the present invention can be manufactured in principle according to synthetic methods known *per se* for nitrooxy organic molecules, and/or based on methods as described in PCT/EP2010/069338, and in PCT/EP2011/072707.

In all these cases appropriate methods to purify the product (compounds of formula II and III) can be chosen by those skilled in the art, i.e. by column chromatography, or the compound of formula (II) or (III), can be isolated and purified by methods known per se, e.g. by adding a solvent such as diethyl-ether or ethyl acetate to induce the separation of the crude product from the mixture after reaction, and drying over Na₂SO₄ of the collected crude product. Preferably, the nitroaniline compound is chosen from the group of compounds according to formula (I) wherein, R1 is H or -CH₃, R2 is H, -CH₃, or -CH₂R3, and R3 is a saturated, linear, or branched C₁-C₃ -hydrocarbon group, and the nitrooxy compound is chosen from the group of compounds according to formula (III) 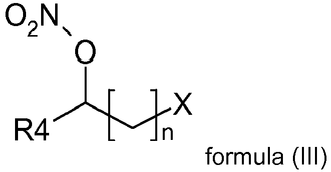 wherein
- n: is comprised between 0 and 6 and, wherein, if n ≠ 0, the carbon chain is a linear, a cyclic, or branched aliphatic carbon chain which may be non-substituted or substituted with up to 3 hydroxyl-, alkoxy-, amino-, alkylamino-, dialkylamino- or nitrooxy groups, or an alkenyl, or an alkynyl carbon chain mono- or polyunsaturated and in any isomeric form,
- R4: is independently, hydrogen or a saturated straight, cyclic or branched chain of an alkyl or alkenyl group containing 1 to 6 carbon atoms,
- X: is hydrogen, R5, -OR5, -OCOR5, -ONO2, -COOR5, -CONR5R6,
- R5 and R6: are independently, hydrogen, C1-C12 straight, branched or cyclic alkyl chain, non-substituted or substituted with up to 3 hydroxyl-, alkoxy-, amino-, alkylamino-, dialkylamino- or nitrooxy groups, alkenyl, or alkynyl carbon chain which may be mono or polyunsaturated, and in any isomeric form.

Even more preferred for the present invention, the nitroaniline compound is selected from the group of compounds comprising: (4-nitro-phenyl)-amine, methyl-(4-nitrophenyl)-amine, ethyl-(4-nitro-phenyl)-amine, dimethyl-(4-nitro-phenyl)-amine, (4-Nitro-phenyl)-propyl-amine, isopropyl-(4-nitro-phenyl)-amine, 2-(4-Nitro-phenylamino)-ethanol, 3-(4-Nitro-phenylamino)-propan-1-ol, 4-Nitro-N-(Pyridin-2-ylmethyl)aniline, 4-Nitro-N-(Pyridin-2-ylmethyl)aniline, and 3-((4-Nitro-phenylamino)-methyl)-benzoic acid, and the nitrooxy compound is selected from 3-Nitrooxypropanol, racemate-4-Phenylbutane-1,2-diyl dinitrate, 2-(Hydroxymethyl)-2-(nitrooxymethyl)-1,3-propanediol, N-Ethyl-3-nitrooxy-propionic sulfonyl amide, 5-Nitrooxy-pentanenitrile, 5-Nitrooxy-pentane, 3-Nitrooxy-propyl propionate, 1,3-bis-Nitrooxypropane, 1,4-bis-Nitrooxybutane, 1,5-bis-Nitrooxypentane, 3-Nitrooxy-propyl benzoate, 3-Nitrooxy-propyl hexanoate, 3-Nitrooxy-propyl 5-nitrooxy-hexanoate, Benzylnitrate, isosorbid-dinitrate, N-[2-(Nitrooxy)ethyl]-3-pyridinecarboxamide, 2-Nitro-5-nitrooxymethyl-furan, Bis-(2-nitrooxyethyl) ether, 3-nitrooxy propionic acid, methyl-3-nitrooxy propionate, Ethyl-3-nitrooxy propionate, Ethyl-4-nitrooxy butanoate, Ethyl-3-nitrooxy butanoate, 5-nitrooxy pentanoic acid, Ethyl-5-nitrooxy pentanoate, 6-nitrooxy hexanoic acid, Ethyl-6-nitrooxy hexanoate, ethyl-4-nitrooxy-cyclohexylcarboxylate, 8-nitrooxy octanoic acid, Ethyl-8-nitrooxy octanoate, 11-nitrooxy undecanoic acid, Ethyl-11-nitrooxy undecanoate, 5-nitrooxy-pentanoic amide, and 5-nitrooxy-N-methyl-pentanoic amide.

Preferably, the present invention provides the use of a the feed composition or feed additive as well as a feed composition or a feed additive comprising a combination of at least one nitroaniline compound and at least one nitrooxy compound as specified in the following list, wherein the compound are individually defined in their respective compound identifier tables (Table 1 (by numbers) and Table 2 (by letters)): 1A, 1B, 1C, 1D, 1E, 1F, 1G, 1H, 1I, 1J, 1K, 1L, 1M, 1N, 1O, 1P, 1Q, 1R, 1S, 1T, 1U, 1V, 1W, 1X, 1Y, 1Z, 1 (AA), 1(AB), 1 (AC), 1(AD), 1(AE), 1 (AF), 2A, 2B, 2C, 2D, 2E, 2F, 2G, 2H, 2I, 2J, 2K, 2L, 2M, 2N, 20, 2P, 2Q, 2R, 2S, 2T, 2U, 2V, 2W, 2X, 2Y, 2Z, 2(AA), 2(AB), 2(AC), 2(AD), 2(AE), 2(AF), 3A, 3B, 3C, 3D, 3E, 3F, 3G, 3H, 3I, 3J, 3K, 3L, 3M, 3N, 30, 3P, 3Q, 3R, 3S, 3T, 3U, 3V, 3W, 3X, 3Y, 3Z, 3(AA), 3(AB), 3(AC), 3(AD), 3(AE), 3(AF), 4A, 4B, 4C, 4D, 4E, 4F, 4G, 4H, 4I, 4J, 4K, 4L, 4M, 4N, 4O, 4P, 4Q, 4R, 4S, 4T, 4U, 4V, 4W, 4X, 4Y, 4Z, 4(AA), 4(AB), 4(AC), 4(AD), 4(AE), 4(AF), 5A, 5B, 5C, 5D, 5E, 5F, 5G, 5H, 5I, 5J, 5K, 5L, 5M, 5N, 5O, 5P, 5Q, 5R, 5S, 5T, 5U, 5V, 5W, 5X, 5Y, 5Z, 5(AA), 5(AB), 5(AC), 5(AD), 5(AE), 5(AF), 6A, 6B, 6C, 6D, 6E, 6F, 6G, 6H, 6I, 6J, 6K, 6L, 6M, 6N, 60, 6P, 6Q, 6R, 6S, 6T, 6U, 6V, 6W, 6X, 6Y, 6Z, 6(AA), 6(AB), 6(AC), 6(AD), 6(AE), 6(AF), 7A, 7B, 7C, 7D, 7E, 7F, 7G, 7H, 7I, 7J, 7K, 7L, 7M, 7N, 70, 7P, 7Q, 7R, 7S, 7T, 7U, 7V, 7W, 7X, 7Y, 7Z, 7(AA), 7(AB), 7(AC), 7(AD), 7(AE), 7(AF), 8A, 8B, 8C, 8D, 8E, 8F, 8G, 8H, 8I, 8J, 8K, 8L, 8M, 8N, 8O, 8P, 8Q, 8R, 8S, 8T, 8U, 8V, 8W, 8X, 8Y, 8Z, 8(AA), 8(AB), 8(AC), 8(AD), 8(AE), 8(AF), 9A, 9B, 9C, 9D, 9E, 9F, 9G, 9H, 9I, 9J, 9K, 9L, 9M, 9N, 90, 9P, 9Q, 9R, 9S, 9T, 9U, 9V, 9W, 9X, 9Y, 9Z, 9(AA), 9(AB), 9(AC), 9(AD), 9(AE), 9(AF), 10A, 10B, 10C, 10D, 10E, 10F, 10G, 10H, 10I, 10J, 10K, 10L, 10M, 10N, 10O, 10P, 10Q, 10R, 10S, 10T, 10U, 10V, 10W, 10X, 10Y, 10Z, 10(AA), 10(AB), 10(AC), 10(AD), 10(AE), 10(AF), 11A, 11B, 11C, 11D, 11E, 11F, 11G, 11H, 11I, 11J, 11K, 11L, 11M, 11N, 11O, 11P, 11Q, 11R, 11S, 11T, 11U, 11V, 11W, 11X, 11Y, 11Z, 11(AA), 11(AB), 11(AC), 11(AD), 11(AE), or 11(AF).

More preferred combination according to the present invention comprises at least one nitroaniline compound selected from the group of compounds comprising: (4-nitro-phenyl)-amine, methyl-(4-nitro-phenyl)-amine, ethyl-(4-nitro-phenyl)-amine, dimethyl-(4-nitro-phenyl)-amine, (4-Nitro-phenyl)-propyl-amine, isopropyl-(4-nitrophenyl)-amine, 2-(4-Nitro-phenylamino)-ethanol, 3-(4-Nitro-phenylamino)-propan-1-ol, 4-Nitro-N-(Pyridin-2-ylmethyl)aniline, 4-Nitro-N-(Pyridin-2-ylmethyl)aniline, and 3-((4-Nitro-phenylamino)-methyl)-benzoic acid, and at least one nitrooxy compound selected from 3-Nitrooxypropanol, 5-Nitrooxy-pentane, 1,3-bis-Nitrooxypropane, ethyl-3-nitrooxy propionate, methyl-3-nitrooxy propionate, 3-nitrooxy propionic acid, 1,4-bis-Nitrooxybutane, and 1,5-bis-Nitrooxypentane.

Another more preferred combination according to the present invention comprises at least one nitroaniline compound selected from the group of compounds comprising: (4-nitro-phenyl)-amine, methyl-(4-nitro-phenyl)-amine, ethyl-(4-nitro-phenyl)-amine, dimethyl-(4-nitro-phenyl)-amine, (4-Nitro-phenyl)-propyl-amine, isopropyl-(4-nitrophenyl)-amine, 2-(4-Nitro-phenylamino)-ethanol, 3-(4-Nitro-phenylamino)-propan-1-ol, 4-Nitro-N-(Pyridin-2-ylmethyl)aniline, 4-Nitro-N-(Pyridin-2-ylmethyl)aniline, and 3-((4-Nitro-phenylamino)-methyl)-benzoic acid, and at least one nitrooxy compound selected from 3-Nitrooxypropanol, and/or 1,3-bis-Nitrooxypropane.

Another more preferred combination according to the present invention comprises at least (4-nitro-phenyl)-amine, and at least one nitrooxy compound selected from: 3-Nitrooxypropanol, racemate-4-Phenylbutane-1,2-diyl dinitrate, 2-(Hydroxymethyl)-2-(nitrooxymethyl)-1,3-propanediol, N-Ethyl-3-nitrooxy-propionic sulfonyl amide, 5-Nitrooxy-pentanenitrile, 5-Nitrooxy-pentane, 3-Nitrooxy-propyl propionate, 1,3-bis-Nitrooxypropane, 1,4-bis-Nitrooxybutane, 1,5-bis-Nitrooxypentane, 3-Nitrooxy-propyl benzoate, 3-Nitrooxy-propyl hexanoate, 3-Nitrooxy-propyl 5-nitrooxy-hexanoate, Benzylnitrate, isosorbid-dinitrate, N-[2-(Nitrooxy)ethyl]-3-pyridinecarboxamide, 2-Nitro-5-nitrooxymethyl-furan, Bis-(2-nitrooxyethyl) ether, 3-nitrooxy propionic acid, methyl-3-nitrooxy propionate, Ethyl-3-nitrooxy propionate, Ethyl-4-nitrooxy butanoate, Ethyl-3-nitrooxy butanoate, 5-nitrooxy pentanoic acid, Ethyl-5-nitrooxy pentanoate, 6-nitrooxy hexanoic acid, Ethyl-6-nitrooxy hexanoate, ethyl-4-nitrooxy-cyclohexylcarboxylate, 8-nitrooxy octanoic acid, Ethyl-8-nitrooxy octanoate, 11-nitrooxy undecanoic acid, Ethyl-11-nitrooxy undecanoate, 5-nitrooxy-pentanoic amide, and 5-nitrooxy-N-methyl-pentanoic amide.

Most preferred combination according to the present invention comprises at least (4-nitro-phenyl)-amine, and at least one nitrooxy compound selected from: 3-Nitrooxypropanol, 5-Nitrooxy-pentane, 1,3-bis-Nitrooxypropane, ethyl-3-nitrooxy propionate, methyl-3-nitrooxy propionate, 3-nitrooxy propionic acid, 1,4-bis-Nitrooxybutane, and 1,5-bis-Nitrooxypentane.

Most preferred combination according to the present invention comprises 3-Nitrooxypropanol and 2-(4-Nitro-phenylamino)-ethanol, or 3-Nitrooxypropanol and (4-Nitro-phenyl)-propyl-amine, or 3-Nitrooxypropanol and (4-nitro-phenyl)-amine, or 1,3-bis-nitrooxypropane and (4-nitro-phenyl)-amine.

Even most preferred is a feed composition or feed additive according to the present invention comprising (4-nitro-phenyl)-amine, and 3-Nitrooxypropanol and /or 1,3-bis-nitrooxypropane.

Methane emission by ruminants can easily be measured in individual animals in metabolic chambers by methods known in the art (Grainger et al., 2007 J. Dairy Science; 90: 2755-2766). Moreover, it can also be assessed at barn level by an emerging technology using laser beam (McGinn et al., 2009, Journal of Environmental Quality; 38: 1796-1802). Alternatively, methane produced by a dairy ruminant can also be assessed by measurement of fatty acid profiles in milk according to WO 2009/156453.

Ruminant performance can be assessed by methods well known in the art, and is usually characterized by feed conversion ratio, feed intake, weight gain, carcass yield, or milk yield.

Further components could be given together with the feed composition or feed additive according to the present invention such as for example yeasts, oregano extracts, and essential oils e.g. thymol, 3-methylphenol, vaniline, guajacol and eugenol.

Ruminating mammals according to the present invention include cattle, goats, sheep, giraffes, American Bison, European bison, yaks, water buffalo, deer, camels, alpacas, Ilamas, wildebeest, antelope, pronghorn, and nilgai.

For all embodiments of the present invention, domestic cattle, sheep and goat are the more preferred species. For the present purposes most preferred species are domestic cattle. The term includes all races of domestic cattle, and all production kinds of cattle, in particular dairy cows and beef cattle.

The present invention also relates to the use of a feed composition or feed additive comprising at least one nitroaniline compound and at least one nitrooxy compound for reducing the formation of methane emanating from the digestive activities of ruminants and/or for improving ruminant performance wherein, the methane production in ruminants, calculated in liters per kilogram of dry matter intake, is reduced by at least 10 % when measured in metabolic chambers.

Preferably, methane reduction is at least 15%, more preferably, at least 20%, even more preferably, at least 25%, most preferably, at least 30%. Alternative methane emission measurements may also be used like using a laser beam or for dairy ruminants, correlating methane production to the VFA profile in milk.

The present invention also relates to the use of a feed composition or feed additive comprising at least one nitroaniline compound and at least one nitrooxy compound, to reduce the ruminant feed conversion ratio by at least 1 % when measured in conventional performance trial. Preferably, the feed conversion ratio is reduced by at least 2%, more preferably, by at least 2.5%, even more preferably, by at least 3%, most preferably, by at least 3.5%.

In animal husbandry, the term feed conversion ratio (FCR), is a measure of an animal's efficiency in converting feed mass into increased body mass. Specifically FCR is the mass of the feed eaten divided by the body mass gain, all over a specified period of time. FCR is dimensionless.

The present invention also relates to the use of a feed composition or feed additive comprising at least one nitroaniline compound, and at least one nitrooxy compound, wherein the amount of the nitroaniline compound in the composition is from 0.1 mg to 2 g per Kg of feed, preferably from 0.1 mg to 0.2 g per Kg of feed, more preferably, from 1 mg to 10 mg per Kg of feed, and the amount of the nitrooxy compound in the composition is from 1 mg to 10 g per Kg of feed, preferably from 5 mg to 1 g per Kg of feed.

The present invention further relates to the use of a feed composition or feed additive comprising at least one nitroaniline compound, and at least one nitrooxy compound, wherein the weight ratio of the nitroaniline compound to the nitrooxy compound is between 0.02 and 0.2, preferably between 0.05 and 0.1.

Ruminant feed or feed additives may be prepared by methods known per se in the art of feed formulation and processing.

Further aspects of the present invention are therefore formulations, i.e. feed additives and animal feed compositions containing compositions as herein above defined.

The present invention therefore also relates to a feed composition or a feed additive comprising at least one nitroaniline compound and at least one nitrooxy compound as defined above.

The normal daily dosage of a composition according to the invention provided to an animal by feed intake depends upon the kind of animal and its condition. Normally this dosage should be in the range of from about 1 mg to about 10 g, preferably from about 10 mg to about 1 g, more preferably, 50 mg to 500 mg compound per kg of body weight.

The composition comprising at least one nitroaniline compound and at least one nitrooxy compound may be used in combination with conventional ingredients present in an animal feed composition (diet) such as calcium carbonates, electrolytes such as ammonium chloride, proteins such as soya bean meal, wheat, starch, sunflower meal, corn, meat and bone meal, amino acids, animal fat, vitamins and trace minerals.

Particular examples of compositions of the invention are the following:
- An animal feed additive comprising (a) at least one compound selected from table 1 and (b) at least one compound selected from table 2, (c) at least one fat-soluble vitamin, (d) at least one water-soluble vitamin, (e) at least one trace mineral, and/or (f) at least one macro mineral;
- An animal feed composition comprising a nitroaniline compound, a nitrooxy compound, and a crude protein content of 50 to 800 g/kg feed.

The so-called premixes are examples of animal feed additives of the invention. A premix designates a preferably uniform mixture of one or more micro-ingredients with diluents and/or carrier. Premixes are used to facilitate uniform dispersion of micro-ingredients in a larger mix.

Apart from the active composition of the invention, the premix usually contains at least one fat-soluble vitamin, and/or at least one water soluble vitamin, and/or at least one trace mineral, and/or at least one macro mineral. In other words, a premix according to the present invention comprises at least one nitroaniline compound and at least one nitrooxy compound according to the invention together with at least one additional component selected from the group consisting of fat-soluble vitamins, water-soluble vitamins, trace minerals, and macro minerals.

Macro minerals may also be separately added to the feed. Therefore, in a particular embodiment, the premix comprises the active ingredients of the invention together with at least one additional component selected from the group consisting of fat-soluble vitamins, water-soluble vitamins, and trace-minerals.

The following are non-exclusive lists of examples of these components:
- Examples of fat-soluble vitamins are vitamin A, vitamin D3, vitamin E, and vitamin K, e.g. vitamin K3.
- Examples of water-soluble vitamins are vitamin B12, biotin and choline, vitamin B1, vitamin B2, vitamin B6, niacin, folic acid and panthothenate, e.g. Ca-D-panthothenate.
- Examples of trace minerals are manganese, zinc, iron, copper, iodine, selenium, and cobalt.
- Examples of macro minerals are calcium, phosphorus and sodium.

As regards feed compositions for ruminants such as cows, as well as ingredients thereof, the ruminant diet is usually composed of an easily degradable fraction (named concentrate) and a fiber-rich less readily degradable fraction (named hay, forage, or roughage).

Hay is made of dried grass, legume or whole cereals. Grasses include among others timothy, ryegrasses, fescues. Legumes include among others clover, lucerne or alfalfa, peas, beans and vetches. Whole cereals include among others barley, maize (corn), oat, sorghum. Other forage crops include sugarcane, kales, rapes, and cabbages. Also root crops such as turnips, swedes, mangles, fodder beet, and sugar beet (including sugar beet pulp and beet molasses) are used to feed ruminants. Still further crops are tubers such as potatoes, cassava and sweet potato. Silage is an ensiled version of the fiber-rich fraction (e.g. from grasses, legumes or whole cereals) whereby material with a high water content is treated with a controlled anaerobic fermentation process (naturally-fermented or additive treated).

Concentrate is largely made up of cereals (such as barley including brewers grain and distillers grain, maize, wheat, sorghum), but also often contain protein-rich feed ingredients such as soybean, rapeseed, palm kernel, cotton seed and sunflower. Cows may also be fed total mixed rations (TMR), where all the dietary components, e.g. forage, silage and concentrate, are mixed before serving.

As mentioned above a premix is an example of a feed additive which may comprise the composition according to the invention. It is understood that the composition may be administered to the animal in different other forms. For example the composition can also be included in a bolus that would be placed in the rumen and that would release a defined amount of the active compounds continuously in well defined dosages over a specific period of time. Disclosed herein is a method for reducing the production of methane emanating from the digestive activities of ruminants and/or for improving ruminant animal performance, comprising orally administering a sufficient amount of a feed composition or feed additive comprising at least one para nitroaniline derivative or salts thereof as defined by formula (I), 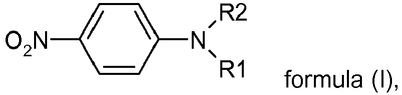 wherein R1 and R2 independently of each other represent H, -CH₃, or - CH₂R3, and wherein R3 is a saturated or unsaturated, linear, branched or cyclic C₁-C₈ -hydrocarbon group, optionally substituted with 1 to 3 groups selected from -OH, -NH₂, -COOH, and wherein one or two of the carbon atoms in the C₁-C₈ hydrocarbon is optionally substituted by a nitrogen or an oxygen atom, and at least one organic molecule substituted at any position with at least one nitrooxy group, or salts thereof as defined by formula (II) 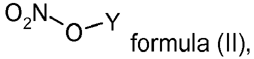 wherein Y is an organic molecule of the following composition: CₐH_{b}O_{d}NₑS_{g}, wherein
- a: is comprised between 1 and 25,
- b: is comprised between 2 and 51,
- d: is comprised between 0 and 8,
- e: is comprised between 0 and 5, and
- g: is comprised between 0 and 3
with the preferred embodiments as described above.

Moreover, the invention further relates to a method as disclosed in the appended claims wherein the feed composition or feed additive according to the present invention is administered to the animal in combination with at least one additional active substance selected from the group consisting of diallyl disulfide, garlic oil, allyl isothiocyanate, deoxycholic acid, chenodeoxycholic acid and derivatives thereof.

The invention also relates to a method as described above, wherein the ruminant animal is selected from the group consisting of: cattle, goats, sheep, giraffes, American Bison, European bison, yaks, water buffalo, deer, camels, alpacas, Ilamas, wildebeest, antelope, pronghorn, and nilgai, and more preferably from the group consisting of: cattle, goats and sheep.

The invention also relates to a method as described above, wherein the amount of the nitroaniline compound in the composition is from 20 mg to 200 g per Kg of feed, preferably from 10 mg to 20 g per Kg of feed, more preferably, from 50 mg to 1 g per Kg of feed, and the amount of nitrooxy compound in the composition of formula is from 1 mg to 10 g per Kg of feed, preferably from 5mg to 1 g per Kg of feed.

The invention also relates to a method as described above, wherein the methane production in ruminants calculated in liters per kilogram of dry matter intake is reduced by at least 10 % when measured in metabolic chambers. Preferably, methane reduction is at least 15%, more preferably, at least 20%, even more preferably, at least 25%, most preferably, at least 30%. Alternative methane emission measurements may also be used like using a laser beam or for dairy ruminants, correlating methane production to the VFA profile in milk.

The invention also relates to a method as described above, wherein the ruminant feed conversion ratio is reduced by at least 1 % when measured in conventional performance trial. Preferably, the feed conversion ratio is reduced by at least 2%, more preferably, by at least 2.5%, even more preferably, by at least 3%, most preferably, by at least 3.5%.

The invention also relates to a method as described above, wherein the weight ratio of the nitroaniline compound to the nitrooxy compound is between 0.02 and 0.2, preferably between 0.05 and 0.1.

The present invention is further described by the following examples which should not be construed as limiting the scope of the invention.

### Examples

### Example 1: In vitro test for methane production.

A modified version of the "Hohenheim Forage value Test (HFT)" was used for testing the effect of specific compounds on the rumen functions mimicked by this in-vitro system.

*Principle:* Feed is gadded into a syringe with a composition of rumen liquor and an appropriate mixture of buffers. The solution is incubated at 39°C. After 8 hours the quantity (and composition) of gas phase produced is measured and put into a formula for conversion.

### Reagents: Mass element solution:

- 6.2 g potassium dihydrogen phosphate (KH₂PO₄)
- 0.6 g magnesium sulfate heptahydrate (MgSO₄ * 7H₂O)
- 9 ml concentrated phosphoric acid (1 mol/l)
- dissolved in distilled water to 1 I (pH about 1.6)

### Buffer solution:

- 35.0 g sodium hydrogen carbonate (NaHCO₃)
- 4.0 g ammonium hydrogen carbonate ((NH₄)HCO₃)
- dissolved in distilled water to 1 l

### Trace element solution:

- 13.2 g calcium chloride dihydrate (Cacl₂ * 2H₂O)
- 10.0 g manganese(II) chloride tetrahydrate (MnCl₂ * 4H₂O)
- 1.0 g cobalt(II) chloride hexahydrate (CoCl₂ * 6H₂O)
- 8.0 g iron(III) chloride (FeCl₃ * 6H₂O)
- dissolved in distilled water to 100 ml

### Sodium salt solution:

- 100 mg sodium salt
- dissolved in distilled water to 100 ml

### Reduction solution:

- first 3 ml sodium hydroxide (c = 1 mol/l), then 427.5 mg sodium sulfide hydrate (Na₂S * H₂O are added to 71.25 ml H₂O
- solution must be prepared shortly before it is added to the medium solution

*Procedure:* Sample weighing: The feed stuff is sieved to 1 mm - usually TMR (44% concentrate, 6% hay, 37% maize silage and 13% grass silage) - and weighed exactly into 64 syringes. 4 of these syringes are the substrate controls, which display the gas production without the effect of the tested compounds. 4 other syringes are positive control, in which bromoethane sulfonate has been added to 0.1 mM. When needed, 4 syringes contain a carrier control (if the test compounds need a carrier). The remaining syringes contain the test substances, by groups of 4 syringes.

### Preparation of the medium solution:

The components are mixed in a Woulff bottle in following order:
- 711 ml water
- 0.18 ml trace element solution
- 355.5 ml buffer solution
- 355.5 ml mass element solution

The completed solution is warmed up to 39°C followed by the addition of 1.83 ml sodium salt solution and the addition of reduction solution at 36°C.

The rumen liquor is added, when the indicator turns colorless.

### Extraction of the rumen liquor:

750 ml of rumen liquor are added to approximately 1,400 ml of medium solution under continued agitation and CO₂-gassing.

### Filling the syringes, incubation and determining gas volumes and VFA values:

The diluted rumen fluid (24 ml) is added to the glass syringe. The syringes are then incubated for 8 h at 39 °C under gentle agitation. After 8 h, the volume of gas produced is measured, and the percentage of methane in the gas phase is determined by gas chromatography.

*Results:* The food fermented was artificial TMR (44% concentrate, 6% hay, 37% maize silage and 13% grass silage). (4-nitro-phenyl)-amine was synthesized as described in PCT/IB2013052230, and used at a concentration of 0.1 % dry matter (DM). 3-nitrooxy-propanol and 1,3-bis-nitrooxypropane were both synthesized as described in PCT/EP2011/072707, and were used at a concentration of 0.005 % DM.

The results are presented in the following Table 3. Surprisingly, synergistic effects were obtained regarding the reduction of methane production. Moreover the acetate, propionate, and butyrate unchanged by the individual doses of the compounds (para nitroalinine and Nitrooxy derivative) are strongly shifted from acetate towards propionate in the case of the composition according to the present invention ((4-nitro-phenyl)-amine together with either 3-nitrooxy-propanol or 1,3-bis-nitrooxypropane. This shift in acetate/propionate ratio translates into additional performance benefit for the animal.

**Table 3:** Effect on Methane reduction, volatile fatty acids (VFA) production and VFA profile resulting from the average of three experiments with ((4-nitro-phenyl)-amine, 3-nitrooxy propanol, and 1,3-bis-nitrooxypropane or combination of both (4-nitrophenyl)-amine, and 3-nitrooxy propanol, or (4-nitro-phenyl)-amine and 1,3-bis-nitrooxypropane . ace. = acetate; Prop. = propionate; But = butyrate; nc = no significant change.

| Composition | Dose (% DM) | Effect on Methanogenesis (% compared to control) | Effect on VFA production (% compared to control) | VFA profile (as percentage of total VFA) | | |
|---|---|---|---|---|---|---|
| | | | | ace. | Prop. | But. |
| (4-nitro-phenyl)-amine | 0.1 % DM | -15% | nc | 59 | 33 | 8 |
| 3-nitrooxy propanol | 0.005 % DM | -9% | nc | 59 | 34 | 6 |
| 1,3-bis-nitrooxypropane | 0.005 % DM | -9% | nc | 59 | 34 | 6 |
| (4-nitro-phenyl)-amine + 3-nitrooxy propanol | 0.1 % DM + 0.005 % DM | -79% | nc | 51 | 41 | 7 |
| (4-nitro-phenyl)-amine + 1,3-bis-nitrooxypropane | 0.1 % DM + 0.005 % DM | -78% | nc | 51 | 41 | 8 |

**Example 2:** *In vitro* reduction of methane production using different combinations of nitroaniline derivatives and nitrooxy derivatives.

In vitro methane reduction experiments were performed as described above in example 1, but the dose is now calculated in molarity (micro molar). The results with different combinations are presented in the following Table 4. Surprisingly, synergistic effects were obtained regarding the reduction of methane production. When combining 3-nitrooxy propanol with either 2-(4-Nitro-phenylamino)-ethanol or (4-Nitro-phenyl)-propyl-amine. Moreover the acetate, propionate, and butyrate unchanged by the individual doses of the compounds (para nitroalinine and Nitrooxy derivative) are strongly shifted from acetate towards propionate in the case of the composition according to the present invention. (data not shown).

**Table 4:** Effect on Methane reduction, and total volatile fatty acids (VFA) production resulting from the average of three experiments with (2-(4-Nitro-phenylamino)-ethanol, (4-Nitro-phenyl)-propyl-amine, 3-nitrooxy propanol, and combination of either of the first two with 3-nitrooxy propanol.

| Composition | Dose (µM) | Effect on Methanogenesis (% compared to control) | Effect on VFA production (% compared to control) |
|---|---|---|---|
| | | | |
| 2-(4-Nitro-phenylamino)-ethanol | 75 | -12% | -4 |
| (4-Nitro-phenyl)-propyl-amine | 75 | -11% | -2 |
| 3-nitrooxy propanol | 0.625 | -8% | -2 |
| 2-(4-Nitro-phenylamino)-ethanol + 3-nitrooxy propanol | 75 + 0.625 | -63% | -7 |
| (4-Nitro-phenyl)-propyl-amine + 3-nitrooxy propanol | 75 + 0.625 | -85% | -10 |

## Claims

1. Use of a feed composition or feed additive comprising at least one para nitroaniline derivative or a salt thereof as defined by formula (I), 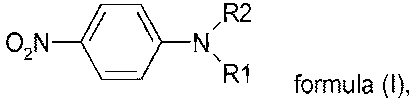 wherein R1 and R2 independently of each other represent H, -CH₃, or -CH₂R3, and wherein R3 is a saturated or unsaturated, linear, branched or cyclic C₁-C₈-hydrocarbon group, optionally substituted with 1 to 3 groups selected from -OH, - NH₂, -COOH, and wherein one or two of the carbon atoms in the C₁-C₈ hydrocarbon is optionally substituted by a nitrogen or an oxygen atom, and at least one organic molecule substituted at any position with at least one nitrooxy group, or a salt thereof as defined by formula (III), 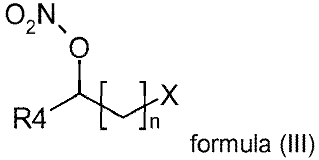 wherein
n is comprised between 0 and 6 and, wherein, if n ≠ 0, the carbon chain is a linear, a cyclic, or branched aliphatic carbon chain which may be non-substituted or substituted with up to 3 hydroxyl-, alkoxy-, amino-, alkylamino-, dialkylamino- or nitrooxy groups, or an alkenyl, or an alkynyl carbon chain mono- or polyunsaturated and in any isomeric form,
R4 is independently, hydrogen or a saturated straight, cyclic or branched chain of an alkyl or alkenyl group containing 1 to 6 carbon atoms,
X is hydrogen, R5, -OR5, -OCOR5, -ONO2, -COOR5, -CONR5R6,
R5 and R6 are independently, hydrogen, C1-C12 straight, branched or cyclic alkyl chain, non-substituted or substituted with up to 3 hydroxyl-, alkoxy-, amino-, alkylamino-, dialkylamino- or nitrooxy groups, alkenyl, or alkynyl carbon chain which may be mono or polyunsaturated, and in any isomeric form.
for reducing the formation of methane emanating from the digestive activities of ruminants and/or for improving ruminant performance and wherein the amount of compound(s) of formula (I) in the feed composition is from 0.1 mg to 0.2 g per kg feed, and the amount of compound(s) of formula (III) in the feed composition is from 1 mg to 10 g per kg feed.

2. The use according to claim 1, wherein
R1 is H or -CH₃,
R2 is H, -CH₃, or -CH₂R3,
R3 is a saturated, linear, or branched C₁-C₃-hydrocarbon group.

3. The use according to claim 1, wherein the compound of formula (I) is selected from the group of compounds comprising: (4-nitro-phenyl)-amine, methyl-(4-nitrophenyl)-amine, ethyl-(4-nitro-phenyl)-amine, dimethyl-(4-nitro-phenyl)-amine, (4-Nitro-phenyl)-propyl-amine, isopropyl-(4-nitro-phenyl)-amine, 2-(4-Nitro-phenylamino)-ethanol, 3-(4-Nitro-phenylamino)-propan-1-ol, 4-Nitro-N-(Pyridin-2-ylmethyl)aniline, 4-Nitro-N-(Pyridin-2-ylmethyl)aniline, and 3-((4-Nitro-phenylamino)-methyl)-benzoic acid, and the compound of formula (III) is selected from 3-Nitrooxypropanol, racemate-4-Phenylbutane-1,2-diyl dinitrate, 2-(Hydroxymethyl)-2-(nitrooxymethyl)-1,3-propanediol, N-Ethyl-3-nitrooxy-propionic sulfonyl amide, 5-Nitrooxy-pentanenitrile, 5-Nitrooxy-pentane, 3-Nitrooxy-propyl propionate, 1,3-bis-Nitrooxypropane, 1,4-bis-Nitrooxybutane, 1,5-bis-Nitrooxypentane, 3-Nitrooxy-propyl benzoate, 3-Nitrooxy-propyl hexanoate, 3-Nitrooxy-propyl 5-nitrooxy-hexanoate, Benzylnitrate, isosorbid-dinitrate, N-[2-(Nitrooxy)ethyl]-3-pyridinecarboxamide, 2-Nitro-5-nitrooxymethyl-furan, Bis-(2-nitrooxyethyl) ether, 3-nitrooxy propionic acid, methyl-3-nitrooxy propionate, Ethyl-3-nitrooxy propionate, Ethyl-4-nitrooxy butanoate, Ethyl-3-nitrooxy butanoate, 5-nitrooxy pentanoic acid, Ethyl-5-nitrooxy pentanoate, 6-nitrooxy hexanoic acid, Ethyl-6-nitrooxy hexanoate, ethyl-4-nitrooxy-cyclohexylcarboxylate, 8-nitrooxy octanoic acid, Ethyl-8-nitrooxy octanoate, 11-nitrooxy undecanoic acid, Ethyl-11-nitrooxy undecanoate, 5-nitrooxy-pentanoic amide, and 5-nitrooxy-N-methyl-pentanoic amide.

4. The use according to claim 1, wherein the compound of formula (I) is selected from the group of compounds comprising: (4-nitro-phenyl)-amine, methyl-(4-nitrophenyl)-amine, ethyl-(4-nitro-phenyl)-amine, dimethyl-(4-nitro-phenyl)-amine, (4-Nitro-phenyl)-propyl-amine, isopropyl-(4-nitro-phenyl)-amine, 2-(4-Nitro-phenylamino)-ethanol, 3-(4-Nitro-phenylamino)-propan-1-ol, 4-Nitro-N-(Pyridin-2-ylmethyl)aniline, 4-Nitro-N-(Pyridin-2-ylmethyl)aniline, and 3-((4-Nitro-phenylamino)-methyl)-benzoic acid, and wherein the organic molecule of formula (III) is 3-nitrooxy propanol and/or 1,3-bis-nitrooxypropane.

5. The use according to claim 1, wherein the compound of formula (I) is (4-nitrophenyl)-amine, and the compound of formula (III) is 3-nitrooxy propanol and/or 1,3-bis-nitrooxypropane.

6. The use according to any of claims 1 to 5, wherein the ruminant animal is selected from the group consisting of: cattle, goats, sheep, giraffes, American Bison, European bison, yaks, water buffalo, deer, camels, alpacas, Ilamas, wildebeest, antelope, pronghorn, and nilgai.

7. The use according to any of claims 1 to 6, wherein the methane production in ruminants calculated in liters per kilogram of dry matter intake is reduced by at least 10 % when measured in metabolic chambers.

8. The use according to any of claims 1 to 7, wherein the weight ratio of the compound(s) of formula (I) to the compound(s) of formula (III) is between 0.02 and 0.2.

9. A feed composition or feed additive comprising at least one para nitroaniline derivative or a salt thereof as defined in formula (I) and at least one organic molecule or a salt thereof of formula (III) as defined in any of claims 1 to 5.

10. The composition of claim 9 which is a mineral premix, a vitamin premix, or a premix including vitamins and minerals, or a bolus.

11. A method for reducing the production of methane emanating from the digestive activities of ruminants and/or for improving ruminant animal performance comprising orally administering to the animal a sufficient amount of a feed composition or feed additive comprising at least one para nitroaniline derivative or salts thereof as defined by formula (I), 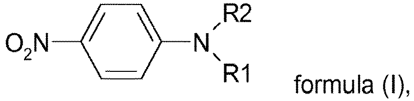 wherein R1 and R2 independently of each other represent H, -CH₃, or -CH₂R3, and wherein R3 is a saturated or unsaturated, linear, branched or cyclic C₁-C₈-hydrocarbon group, optionally substituted with 1 to 3 groups selected from -OH, - NH₂, -COOH, and wherein one or two of the carbon atoms in the C₁-C₈ hydrocarbon is optionally substituted by a nitrogen or an oxygen atom, and at least one organic molecule substituted at any position with at least one nitrooxy group, or salts thereof as defined by formula (III) 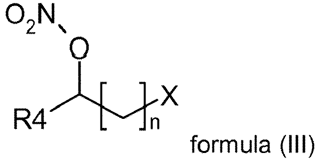 wherein
n is comprised between 0 and 6 and, wherein, if n ≠ 0, the carbon chain is a linear, a cyclic, or branched aliphatic carbon chain which may be non-substituted or substituted with up to 3 hydroxyl-, alkoxy-, amino-, alkylamino-, dialkylamino- or nitrooxy groups, or an alkenyl, or an alkynyl carbon chain mono- or polyunsaturated and in any isomeric form,
R4 is independently, hydrogen or a saturated straight, cyclic or branched chain of an alkyl or alkenyl group containing 1 to 6 carbon atoms,
X is hydrogen, R5, -OR5, -OCOR5, -ONO2, -COOR5, -CONR5R6,
R5 and R6 are independently, hydrogen, C1-C12 straight, branched or cyclic alkyl chain, non-substituted or substituted with up to 3 hydroxyl-, alkoxy-, amino-, alkylamino-, dialkylamino- or nitrooxy groups, alkenyl, or alkynyl carbon chain which may be mono or polyunsaturated, and in any isomeric form.
wherein the amount of compound(s) of formula (I) in the feed composition is from 0.1 mg to 0.2 g per kg feed, and the amount of compound(s) of formula (III) in the feed composition is from 1 mg to 10 g per kg feed.

12. A method according to claim 11, wherein the ruminant animal is selected from the group consisting of: cattle, goats, sheep, giraffes, American Bison, European bison, yaks, water buffalo, deer, camels, alpacas, Ilamas, wildebeest, antelope, pronghorn, and nilgai.

13. A method according to any of claims 11 or 12, wherein the weight ratio of the compound(s) of formula (I) to the compound(s) of formula (III) is between 0.02 and 0.2.

## Patentansprüche

1. Verwendung einer Futtermittelzusammensetzung oder eines Futtermittelzusatzstoffs, umfassend mindestens ein para-Nitroanilinderivat oder ein Salz davon wie in Formel (I) definiert, 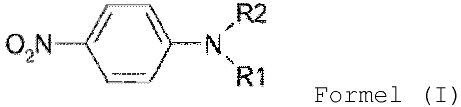 wobei R1 und R2 unabhängig voneinander H, -CH₃ oder -CH₂R3 bedeuten und wobei R3 eine gesättigte oder ungesättigte geradkettige, verzweigte oder cyclische C₁-C₈-Kohlenwasserstoffgruppe bedeutet, die gegebenenfalls durch 1 bis 3 Gruppen, ausgewählt aus -OH, -NH₂, -COOH, substituiert ist, und wobei eines oder zwei der Kohlenstoffatome in dem C₁-C₈-Kohlenwasserstoff gegebenenfalls durch ein Stickstoff- oder ein Sauerstoffatom substituiert ist, sowie mindestens ein organisches Molekül, das an einer beliebigen Position mit mindestens einer Nitrooxygruppe substituiert ist, oder ein Salz davon wie in Formel (III) definiert, 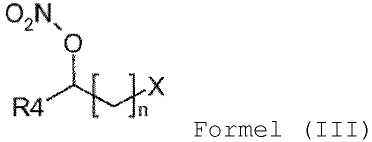 wobei
n im Bereich von 0 bis 6 liegt und wobei, falls n ≠ 0, die Kohlenstoffkette eine geradkettige, eine cyclische oder eine verzweigte aliphatische Kohlenstoffkette ist, die unsubstituiert oder durch bis zu 3 Hydroxyl-, Alkoxy-, Amino-, Alkylamino-, Dialkylamino- oder Nitrooxygruppen substituiert sein kann, oder ein Alkenyl, oder eine Alkinylkohlenstoffkette, einfach oder mehrfach ungesättigt und in einer beliebigen isomeren Form sein kann,
R4 unabhängig Wasserstoff oder eine gesättigte geradkettige, cyclische oder verzweigte Kette einer Alkyl- oder Alkenylgruppe mit 1 bis 6 Kohlenstoffatomen bedeutet,
X Wasserstoff, R5, -OR5, -OCOR5, -ONO2, - COOR5, -CONR5R6 bedeutet,
R5 und R6 unabhängig Wasserstoff, eine geradkettige, verzweigte oder cyclische C1-C12-Alkylkette, unsubstituiert oder substituiert durch bis zu 3 Hydroxyl-, Alkoxy-, Amino-, Alkylamino-, Dialkylamino- oder Nitrooxygruppen, Alkenyl, oder eine Alkinylkohlenstoffkette, die einfach oder mehrfach ungesättigt sein kann, in einer beliebigen isomeren Form, bedeuten,
zum Vermindern der Bildung des Methans, das aufgrund der Verdauungsaktivitäten von Wiederkäuern entsteht, und/oder zum Verbessern der Leistung von Wiederkäuern, und wobei die Menge an Verbindung(en) der Formel (I) in der Futtermittelzusammensetzung 0,1 mg bis 0,2 g pro kg Futtermittel beträgt und die Menge an Verbindung (en) der Formel (III) in der Futtermittelzusammensetzung 1 mg bis 10 g pro kg Futtermittel beträgt.

2. Verwendung nach Anspruch 1, wobei
R1 H oder -CH₃ bedeutet,
R2 H, -CH₃ oder -CH₂R3 bedeutet,
R3 eine gesättigte geradkettige oder verzweigte C₁-C₃-Kohlenwasserstoffgruppe bedeutet.

3. Verwendung nach Anspruch 1, wobei die Verbindung der Formel (I) aus der Gruppe von Verbindungen umfassend die Folgenden ausgewählt ist: (4-Nitrophenyl)amin, Methyl-(4-nitrophenyl)amin, Ethyl-(4-nitrophenyl)amin, Dimethyl-(4-nitrophenyl)amin, (4-Nitrophenyl)propylamin, Isopropyl-(4-nitrophenyl)amin, 2-(4-Nitrophenylamino)ethanol, 3-(4-Nitrophenylamino)propan-1-ol, 4-Nitro-N-(pyridin-2-ylmethyl)anilin, 4-Nitro-N-(pyridin-2-ylmethyl)anilin und 3-((4-Nitrophenyl-amino)methyl)benzoesäure und die Verbindung der Formel (III) aus den Folgenden ausgewählt ist: 3-Nitrooxypropanol, Racemat-4-phenylbutan-1,2-diyldinitrat, 2-(Hydroxymethyl)-2-(nitrooxymethyl)-1,3-propandiol, N-Ethyl-3-nitrooxypropionsäuresulfonylamid, 5-Nitrooxypentannitril, 5-Nitrooxypentan, 3-Nitrooxypropylpropionat, 1,3-bis-Nitrooxypropan, 1,4-bis-Nitrooxybutan, 1,5-bis-Nitrooxypentan, 3-Nitrooxypropylbenzoat, 3-Nitrooxypropylhexanoat, 3-Nitrooxypropyl-5-nitrooxyhexanoat, Benzylnitrat, Isosorbiddinitrat, N-[2-(Nitrooxy)ethyl]-3-pyridincarboxamid, 2-Nitro-5-nitrooxymethylfuran, Bis-(2-nitrooxyethyl)ether, 3-Nitrooxypropionsäure, Methyl-3-nitrooxypropionat, Ethyl-3-nitrooxypropionat, Ethyl-4-nitrooxybutanoat, Ethyl-3-nitrooxybutanoat, 5-Nitrooxypentansäure, Ethyl-5-nitrooxypentanoat, 6-Nitrooxyhexansäure, Ethyl-6-Nitrooxyhexanoat, Ethyl-4-nitrooxycyclohexyl-carboxylat, 8-Nitrooxyoctansäure, Ethyl-8-nitrooxyoctanoat, 11-Nitrooxyundecansäure, Ethyl-11-nitrooxyundecanoat, 5-Nitrooxypentanamid und 5-Nitrooxy-N-methylpentanamid.

4. Verwendung nach Anspruch 1, wobei die Verbindung der Formel (I) aus der Gruppe von Verbindungen umfassend die Folgenden ausgewählt ist: (4-Nitrophenyl)amin, Methyl-(4-nitrophenyl)amin, Ethyl-(4-nitrophenyl)amin, Dimethyl-(4-nitrophenyl)amin, (4-Nitrophenyl)propylamin, Isopropyl-(4-nitrophenyl)amin, 2-(4-Nitrophenylamino)ethanol, 3-(4-Nitrophenylamino)propan-1-ol, 4-Nitro-N-(pyridin-2-ylmethyl)anilin, 4-Nitro-N-(pyridin-2-ylmethyl)anilin und 3-((4-Nitrophenylamino)methyl)benzoesäure, und wobei es sich bei dem organischen Molekül der Formel (III) um 3-Nitrooxypropanol und/oder 1,3-bis-Nitrooxypropan handelt.

5. Verwendung nach Anspruch 1, wobei es sich bei der Verbindung der Formel (I) um (4-Nitrophenyl)amin und bei der Verbindung der Formel (III) um 3-Nitrooxypropanol und/oder 1,3-bis-Nitrooxypropan handelt.

6. Verwendung nach einem der Ansprüche 1 bis 5, wobei der Wiederkäuer aus der Gruppe bestehend aus Rindern, Ziegen, Schafen, Giraffen, Bison, Wisent, Yaks, Wasserbüffel, Hirschen, Kamelen, Alpakas, Lamas, Wildebeest, Antilope, Pronghorn und Nilgai ausgewählt ist.

7. Verwendung nach einem der Ansprüche 1 bis 6, wobei die in Litern pro kg Trockenmasseaufnahme berechnete Methanproduktion bei Wiederkäuern bei Messung in Metabolikkammern um mindestens 10% vermindert ist.

8. Verwendung nach einem der Ansprüche 1 bis 7, wobei das Gewichtsverhältnis der Verbindung(en) der Formel (I) zu der/den Verbindung(en) der Formel (III) zwischen 0,02 und 0,2 beträgt.

9. Futtermittelzusammensetzung oder Futtermittelzusatzstoff, umfassend mindestens ein para-Nitroanilinderivat oder ein Salz davon wie in Formel (I) definiert und mindestens ein organisches Molekül oder ein Salz davon der Formel (III) wie in einem der Ansprüche 1 bis 5 definiert.

10. Zusammensetzung nach Anspruch 9, bei der es sich um eine Mineralstoffvormischung, eine Vitaminvormischung oder eine Vormischung, die Vitamine und Mineralien beinhaltet, oder einen Bolus handelt.

11. Verfahren zum Vermindern der Produktion von Methan, das aufgrund der Verdauungsaktivitäten von Wiederkäuern entsteht, und/oder zum Verbessern der Leistung von Wiederkäuern, umfassend die orale Verabreichung einer ausreichenden Menge einer Futtermittelzusammensetzung oder eines Futtermittelzusatzstoffs, umfassend mindestens ein para-Nitroanilinderivat oder Salze davon wie in Formel (I) definiert, 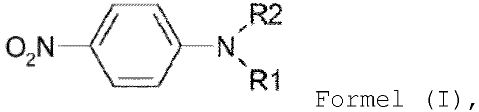 wobei R1 und R2 unabhängig voneinander H, -CH₃ oder -CH₂R3 bedeuten und wobei R3 eine gesättigte oder ungesättigte geradkettige, verzweigte oder cyclische C₁-C₈-Kohlenwasserstoffgruppe, gegebenenfalls substituiert mit 1 bis 3 Gruppen, ausgewählt aus -OH, -NH₂, -COOH, bedeutet und wobei eines oder zwei der Kohlenstoffatome in dem C₁-C₈-Kohlenwasserstoff gegebenenfalls durch ein Stickstoff- oder ein Sauerstoffatom substituiert ist, sowie mindestens ein organisches Molekül, das an einer beliebigen Position mit mindestens einer Nitrooxygruppe substituiert ist, oder Salze davon wie in Formel (III) definiert, 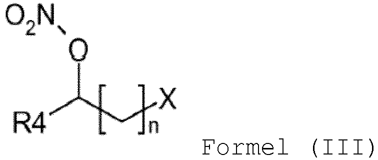 wobei
n im Bereich von 0 bis 6 liegt und wobei, falls n ≠ 0, die Kohlenstoffkette eine geradkettige, eine cyclische oder eine verzweigte aliphatische Kohlenstoffkette ist, die unsubstituiert oder durch bis zu 3 Hydroxyl-, Alkoxy-, Amino-, Alkylamino-, Dialkylamino- oder Nitrooxygruppen substituiert sein kann, oder ein Alkenyl, oder eine Alkinylkohlenstoffkette, einfach oder mehrfach ungesättigt und in einer beliebigen isomeren Form sein kann,
R4 unabhängig Wasserstoff oder eine gesättigte geradkettige, cyclische oder verzweigte Kette einer Alkyl- oder Alkenylgruppe mit 1 bis 6 Kohlenstoffatomen bedeutet,
X Wasserstoff, R5, -OR5, -OCOR5, -ONO2, - COOR5, -CONR5R6 bedeutet,
R5 und R6 unabhängig Wasserstoff, eine geradkettige, verzweigte oder cyclische C1-C12-Alkylkette, unsubstituiert oder substituiert durch bis zu 3 Hydroxyl-, Alkoxy-, Amino-, Alkylamino-, Dialkylamino- oder Nitrooxygruppen, Alkenyl, oder eine Alkinylkohlenstoffkette, die einfach oder mehrfach ungesättigt sein kann, in einer beliebigen isomeren Form, bedeuten,
an ein Tier, wobei die Menge an Verbindung(en) der Formel (I) in der Futtermittelzusammensetzung 0,1 mg bis 0,2 g pro kg Futtermittel beträgt und die Menge an Verbindung (en) der Formel (III) in der Futtermittelzusammensetzung 1 mg bis 10 g pro kg Futtermittel beträgt.

12. Verfahren nach Anspruch 11, wobei der Wiederkäuer aus der Gruppe bestehend aus Rindern, Ziegen, Schafen, Giraffen, Bison, Wisent, Yaks, Wasserbüffel, Hirschen, Kamelen, Alpakas, Lamas, Wildebeest, Antilope, Pronghorn und Nilgai ausgewählt ist.

13. Verfahren nach einem der Ansprüche 11 oder 12, wobei das Gewichtsverhältnis der Verbindung(en) der Formel (I) zu der/den Verbindung(en) der Formel (III) zwischen 0,02 und 0,2 beträgt.

## Revendications

1. Utilisation d'une composition d'aliment pour animaux ou d'un additif pour aliment pour animaux, comprenant au moins un dérivé de para-nitroaniline ou un sel de celui-ci, tel que défini par la formule (I), 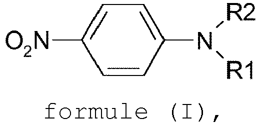 où R1 et R2 représentent indépendamment l'un de l'autre H, -CH₃, ou -CH₂R3, et où R3 est un groupement hydrocarboné en C₁-C₈ saturé ou insaturé, linéaire, ramifié ou cyclique, éventuellement substitué par de 1 à 3 groupements choisis parmi -OH, -NH₂, -COOH, et où un ou deux parmi les atomes de carbone dans le groupement hydrocarboné en C₁-C₈ sont éventuellement substitués par un atome d'azote ou d'oxygène, et
au moins une molécule organique substituée au niveau d'une position quelconque par au moins un groupement nitrooxy, ou un sel de celui-ci, telle que définie par la formule (III), 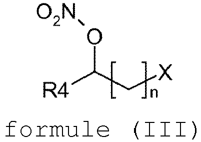 où
n est compris entre 0 et 6, et si n ≠ 0, la chaîne carbonée est une chaîne carbonée aliphatique linéaire, cyclique ou ramifiée, qui peut être non substituée ou substituée par jusqu'à 3 groupements hydroxyl-, alcoxy-, amino-, alkylamino-, dialkylamino- ou nitrooxy, ou une chaîne carbonée alcényle, ou alcynyle mono- ou polyinsaturée et sous une forme isomère quelconque,
R4 est indépendamment hydrogène ou une chaîne saturée linéaire, cyclique ou ramifiée d'un groupement alkyle ou alcényle contenant de 1 à 6 atomes de carbone,
X est hydrogène, R5, -OR5, -OCOR5, -ONO2, - COOR5, -CONR5R6,
R5 et R6 sont indépendamment hydrogène, une chaîne alkyle en C₁-C₁₂ linéaire, ramifiée ou cyclique, non substituée ou substituée par jusqu'à 3 groupements hydroxyl-, alcoxy-, amino-, alkylamino-, dialkylamino- ou nitrooxy, une chaîne carbonée alcényle, ou alcynyle pouvant être mono- ou polyinsaturée, et sous une forme isomère quelconque,
pour la réduction de la formation de méthane émanant des activités digestives de ruminants et/ou pour améliorer les performances des ruminants et où la quantité du ou des composés de formule (I) dans la composition d'aliment pour animaux va de 0,1 mg à 0,2 g par kg d'aliment, et la quantité du ou des composés de formule (III) dans la composition d'aliment pour animaux va de 1 mg à 10 g par kg d'aliment.

2. Utilisation selon la revendication 1, dans laquelle
R1 est H ou -CH₃,
R2 est H, -CH₃ ou -CH₂R3,
R3 est un groupement hydrocarboné en C₁-C₃ saturé, linéaire ou ramifié.

3. Utilisation selon la revendication 1, dans laquelle le composé de formule (I) est choisi dans le groupe constitué par les composés comprenant : la (4-nitrophényl)amine, la méthyl(4-nitrophényl)amine, l'éthyl(4-nitrophényl)amine, la diméthyl(4-nitrophényl)-amine, la (4-nitrophényl)propylamine, l'isopropyl-(4-nitrophényl)amine, le 2-(4-nitrophénylamino)éthanol, le 3-(4-nitrophénylamino)propan-1-ol, la 4-nitro-N-(pyridin-2-ylméthyl)aniline, la 4-nitro-N-(pyridin-2-ylméthyl)aniline, et l'acide 3-((4-nitrophénylamino)-méthyl)benzoïque, et le composé de formule (III) est choisi parmi le 3-nitrooxypropanol, le racémate du dinitrate de 4-phénylbutane-1,2-diyle, le 2-(hydroxyméthyl)-2-(nitrooxyméthyl)-1,3-propanediol, le sulfonylamide N-éthyl-3-nitrooxy-propionique, le 5-nitrooxy-pentanenitrile, le 5-nitrooxy-pentane, le propionate de 3-nitrooxy-propyle, le 1,3-bis-nitrooxypropane, le 1,4-bis-nitrooxybutane, le 1,5-bis-nitrooxypentane, le benzoate de 3-nitrooxy-propyle, l'hexanoate de 3-nitrooxy-propyle, le 5-nitrooxy-hexanoate de 3-nitrooxy-propyle, le nitrate de benzyle, le dinitrate d'isosorbide, le N-[2-(nitrooxy)éthyl]-3-pyridinecarboxamide, le 2-nitro-5-nitrooxyméthyl-furane, le bis-(2-nitrooxyéthyl)éther, l'acide 3-nitrooxy-propionique, le 3-nitrooxy-propionate de méthyle, le 3-nitrooxy-propionate d'éthyle, le 4-nitrooxy-butanoate d'éthyle, le 3-nitrooxy-butanoate d'éthyle, l'acide 5-nitrooxy-pentanoïque, le 5-nitrooxy-pentanoate d'éthyle, l'acide 6-nitrooxy-hexanoïque, le 6-nitrooxy-hexanoate d'éthyle, le 4-nitrooxy-cyclohexylcarboxylate d'éthyle, l'acide 8-nitrooxy-octanoïque, le 8-nitrooxy-octanoate d'éthyle, l'acide 11-nitrooxy-undécanoïque, le 11-nitrooxy-undécanoate d'éthyle, l'amide 5-nitrooxy-pentanoïque, et l'amide 5-nitrooxy-N-méthylpentanoique.

4. Utilisation selon la revendication 1, dans laquelle le composé de formule (I) est choisi dans le groupe constitué par les composés comprenant : la (4-nitrophényl)amine, la méthyl(4-nitrophényl)amine, l'éthyl(4-nitrophényl)amine, la diméthyl(4-nitrophényl)amine, la (4-nitrophényl)propylamine, l'isopropyl(4-nitrophényl)amine, le 2-(4- nitrophénylamino)éthanol, le 3-(4-nitrophénylamino)-propan-1-ol, la 4-nitro-N-(pyridin-2-ylméthyl)aniline, la 4-nitro-N-(pyridin-2-ylméthyl)aniline, et l'acide 3-((4-nitrophénylamino)méthyl)benzoïque, et où la molécule organique de formule (III) est le 3-nitrooxy-propanol et/ou le 1,3-bis-nitrooxypropane.

5. Utilisation selon la revendication 1, dans laquelle le composé de formule (I) est la (4-nitrophényl)amine, et le composé de formule (III) est le 3-nitrooxy-propanol et/ou le 1,3-bis-nitrooxypropane.

6. Utilisation selon l'une quelconque des revendications 1 à 5, dans laquelle l'animal ruminant est choisi dans le groupe constitué par les bovins, les chèvres, les ovins, les girafes, le bison d'Amérique, le bison d'Europe, les yacks, le buffle des Indes, les cervidés, les camélidés, les alpagas, les lamas, les gnous, les antilopes, les antilopes d'Amérique, et le nilgaut.

7. Utilisation selon l'une quelconque des revendications 1 à 6, dans laquelle la production de méthane chez les ruminants, calculée en litres par kilogramme d'apport en matière sèche, est réduite d'au moins 10%, lors d'une mesure dans des chambres métaboliques.

8. Utilisation selon l'une quelconque des revendications 1 à 7, dans laquelle le rapport pondéral du ou des composés de formule (I) au(x) composé (s) de formule (III) est compris entre 0,02 et 0,2.

9. Composition d'aliment pour animaux ou additif pour aliment pour animaux, comprenant au moins un dérivé de para-nitroaniline ou un sel de celui-ci tel que défini dans la formule (I) et au moins une molécule organique ou un sel de celle-ci de formule (III) telle que définie selon l'une quelconque des revendications 1 à 5.

10. Composition selon la revendication 9, qui est un prémélange de minéraux, un prémélange de vitamines, ou un prémélange comportant des vitamines et des minéraux, ou un bolus.

11. Méthode de réduction de la production de méthane émanant des activités digestives de ruminants et/ou pour améliorer les performances d'animaux ruminants, comprenant l'administration par voie orale à l'animal d'une quantité suffisante d'une composition d'aliment pour animaux ou d'un additif pour aliment pour animaux, comprenant au moins un dérivé de para-nitroaniline ou des sels de celui-ci, tel que défini par la formule (I), 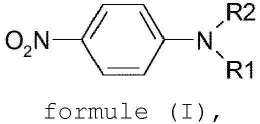 où R1 et R2 représentent indépendamment l'un de l'autre H, -CH₃, ou -CH₂R3, et où R3 est un groupement hydrocarboné en C₁-C₈ saturé ou insaturé, linéaire, ramifié ou cyclique, éventuellement substitué par de 1 à 3 groupements choisis parmi -OH, -NH₂, -COOH, et où un ou deux parmi les atomes de carbone dans le groupement hydrocarboné en C₁-C₈ sont éventuellement substitués par un atome d'azote ou d'oxygène, et
au moins une molécule organique substituée au niveau d'une position quelconque par au moins un groupement nitrooxy, ou des sels de celle-ci, telle que définie par la formule (III) 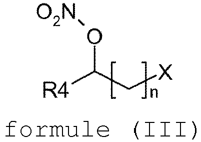 où n est compris entre 0 et 6, et si n ≠ 0, la chaîne carbonée est une chaîne carbonée aliphatique linéaire, cyclique ou ramifiée, qui peut être non substituée ou substituée par jusqu'à 3 groupements hydroxyl-, alcoxy-, amino-, alkylamino-, dialkylamino- ou nitrooxy, ou une chaîne carbonée alcényle, ou alcynyle mono- ou polyinsaturée et sous une forme isomère quelconque,
R4 est indépendamment hydrogène ou une chaîne saturée linéaire, cyclique ou ramifiée d'un groupement alkyle ou alcényle contenant de 1 à 6 atomes de carbone,
X est hydrogène, R5, -OR5, -OCOR5, -ONO2, - COOR5, -CONR5R6,
R5 et R6 sont indépendamment hydrogène, une chaîne alkyle en C₁-C₁₂ linéaire, ramifiée ou cyclique, non substituée ou substituée par jusqu'à 3 groupements hydroxyl-, alcoxy-, amino-, alkylamino-, dialkylamino- ou nitrooxy, une chaîne carbonée alcényle, ou alcynyle pouvant être mono- ou polyinsaturée, et sous une forme isomère quelconque,
où la quantité du ou des composés de formule (I) dans la composition d'aliment pour animaux va de 0,1 mg à 0,2 g par kg d'aliment, et la quantité du ou des composés de formule (III) dans la composition d'aliment pour animaux va de 1 mg à 10 g par kg d'aliment.

12. Méthode selon la revendication 11, dans laquelle l'animal ruminant est choisi dans le groupe constitué par les bovins, les chèvres, les ovins, les girafes, le bison d'Amérique, le bison d'Europe, les yacks, le buffle des Indes, les cervidés, les camélidés, les alpagas, les lamas, les gnous, les antilopes, les antilopes d'Amérique, et le nilgaut.

13. Méthode selon l'une quelconque des revendications 11 ou 12, dans laquelle le rapport pondéral du ou des composés de formule (I) au(x) composé(s) de formule (III) est compris entre 0,02 et 0,2.
